(19) **Europäisches Patentamt**
**European Patent Office**
**Office européen des brevets**

(11) **EP 3 438 124 A1**

(12) **EUROPEAN PATENT APPLICATION**
published in accordance with Art. 153(4) EPC

(43) Date of publication:
06.02.2019 Bulletin 2019/06

(51) Int Cl.:
*C07K 14/78* $^{(2006.01)}$    *A61L 27/00* $^{(2006.01)}$
*C12M 1/00* $^{(2006.01)}$    *C12N 5/07* $^{(2010.01)}$
*C12N 15/09* $^{(2006.01)}$

(21) Application number: 17774879.5

(22) Date of filing: 27.03.2017

(86) International application number:
PCT/JP2017/012284

(87) International publication number:
WO 2017/170342 (05.10.2017 Gazette 2017/40)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**MA MD**

(30) Priority: 29.03.2016 JP 2016065396

(71) Applicants:
• **FUJIFILM Corporation**
**Tokyo 106-8620 (JP)**
• **Terumo Kabushiki Kaisha**
**Tokyo 151-0072 (JP)**

(72) Inventors:
• **NAKAMURA Kentaro**
**Ashigarakami-gun**
**Kanagawa 258-8577 (JP)**
• **SANO Shinya**
**Ashigarakami-gun**
**Kanagawa 259-0151 (JP)**
• **KURUMA Yosuke**
**Ashigarakami-gun**
**Kanagawa 259-0151 (JP)**
• **SAMESHIMA Tadashi**
**Ashigarakami-gun**
**Kanagawa 259-0151 (JP)**

(74) Representative: **Klunker IP Patentanwälte PartG mbB Destouchesstraße 68 80796 München (DE)**

(54) **CELL SHEET EMBEDDING AGENT, CELL SHEET EMBEDDING COMPOSITION AND KIT**

(57) An object of the present invention is to provide a cell sheet embedding agent, which makes it possible to stably transport a cell sheet at the time of transport at a low temperature and to collect the cell sheet from the cell sheet embedding agent in a simple manner after being transported, a cell sheet-containing composition, and a kit. According to the present invention, there is provided a cell sheet embedding agent containing a polypeptide represented by the following Formula 1, in which in a case where a molecular weight distribution of the polypeptide is measured, a peak area of a maximum molecular weight is equal to or greater than 80% of the total peak area of all molecular weights.

Formula 1:      $A-[(Gly-X-Y)_n]_m-B$

In the formula, X and Y each independently represent an amino acid, m is an integer of 2 to 10, n is an integer of 3 to 100, and A and B each represent any amino acid or any amino acid sequence.

**EP 3 438 124 A1**

## FIG. 1

**Description**

**BACKGROUND OF THE INVENTION**

1. Field of the Invention

The present invention relates to a cell sheet embedding agent containing a polypeptide having a molecular weight distribution satisfying a predetermined condition. The present invention also relates to a cell sheet-containing composition and a kit which contain the cell sheet embedding agent.

2. Description of the Related Art

In recent years, regenerative medicine and cell transplantation therapy have been frequently practiced. Particularly, a therapy has been frequently examined in which cells are made into a cell sheet and only the cells are transplanted in the form of a sheet. It is considered that compared to the administration of individual cells as a suspension, the therapy results in better engraftment in the body and further improves the efficacy because the cell sheet is maintained as a structure. Patent Literature 1 aims to provide a manufacturing method using a cell culture solution, which does not contain impurity components resulting from the manufacturing process that hinder the clinical application, and describes a method for manufacturing a cell sheet including culturing cells having a density enabling the formation of a cell sheet substantially without causing growth in a cell culture solution containing no growth factor in an effective amount.

Meanwhile, regarding individual cells not being in the form of a sheet, a method is being examined in which the cells are transplanted by being combined with a scaffolding material that becomes a support of the cells. As the scaffolding material that becomes the support of the cells, gene recombinant gelatin is known. Patent Literature 2 describes gene recombinant gelatin which is useful for several uses accompanying cell adhesion such as a cell culture operation and accompanying the culture of scaffold-dependent cells, and is particularly useful for various medical uses.

Prior Art Literatures

Patent Literatures

Patent Literature 1: JP2010-81829A
Patent Literature 2: WO2008/103041A

**SUMMARY OF THE INVENTION**

Unfortunately, the cell sheet is extremely brittle and easily broken at the time of transport, which results in problems at the time of transport to hospital facilities and the like. Furthermore, in hospital facilities and the like, it is desired that the cell sheet can be transplanted in a simple manner after being transported. For the transplantation of the cell sheet, the above problems need to be solved. An object of the present invention is to provide a cell sheet embedding agent which makes it possible to stably transport a cell sheet at the time of transport at a low temperature and to collect the cell sheet from the cell sheet embedding agent in a simple manner after being transported. Another object of the present invention is to provide a cell sheet-containing composition which contains the cell sheet embedding agent and a kit which contains the cell sheet embedding agent.

In order to achieve the above objects, the inventors of the present invention conducted intensive examinations. As a result, the inventors obtained knowledge that by a cell sheet embedding agent containing a polypeptide which has a specific sequence and in which in a case where a molecular weight distribution of the polypeptide is measured, a peak area of a maximum molecular weight is found to be equal to or greater than 80% of the total peak area of all molecular weights, a cell sheet can be stably transported at the time of transport at a low temperature, and the cell sheet can be collected from the cell sheet embedding agent in a simple manner at room temperature at the time of transplanting the cell sheet after being transported. The present invention has been accomplished based on the knowledge. According to the present invention, the following inventions are provided.

[1] A cell sheet embedding agent comprising a polypeptide which is represented by the following Formula 1 and has a molecular weight distribution satisfying the following condition X.

Formula 1:          $A\text{-}[(Gly\text{-}X\text{-}Y)_n]_m\text{-}B$

In the formula, n X's each independently represent any amino acid, n Y's each independently represent any amino acid, m is an integer of 2 to 10, n is an integer of 3 to 100, A represents any amino acid or any amino acid sequence, and B represents any amino acid or any amino acid sequence.

Condition X: in a case where the molecular weight distribution is measured by gel permeation chromatography, a peak area of a maximum molecular weight is equal to or greater than 80% of the total peak area of all molecular weights.

[2] The cell sheet embedding agent described in [1], in which the polypeptide is recombinant gelatin.

[3] The cell sheet embedding agent described in [1] or [2], in which the polypeptide is a polypeptide represented by the following Formula 2.

Formula 2: $\text{Gly-Ala-Pro-[(Gly-X-Y)}_{63}]_3\text{-Gly}$

In the formula, 63 X's each independently represent any amino acid, 63 Y's each independently represent any amino acid, and 63 sequences represented by Gly-X-Y may be the same as or different from each other.

[4] The cell sheet embedding agent described in any one of [1] to [3], in which the polypeptide has (1) amino acid sequence described in SEQ ID NO: 1 or (2) amino acid sequence which shares a sequence identity equal to or higher than 80% with the amino acid sequence described in SEQ ID NO: 1 and has biocompatibility.

[5] The cell sheet embedding agent described in any one of [1] to [4], in which the polypeptide has the amino acid sequence described in SEQ ID NO: 1.

[6] A cell sheet-containing composition comprising a cell sheet and the cell sheet embedding agent described in any one of [1] to [5], in which the cell sheet is embedded in the cell sheet embedding agent.

[7] A kit comprising a cell sheet and the cell sheet embedding agent described in any one of [1] to [5].

[8] A polypeptide which is used for embedding a cell sheet, represented by the following Formula 1, and has a molecular weight distribution satisfying the following condition X.

Formula 1: $\text{A-[(Gly-X-Y)}_n]_m\text{-B}$

In the formula, n X's each independently represent any amino acid, n Y's each independently represent any amino acid, m is an integer of 2 to 10, n is an integer of 3 to 100, A represents any amino acid or any amino acid sequence, and B represents any amino acid or any amino acid sequence.

Condition X: in a case where the molecular weight distribution is measured by gel permeation chromatography, a peak area of a maximum molecular weight is equal to or greater than 80% of the total peak area of all molecular weights.

[9] Use of a polypeptide which is used for manufacturing a cell sheet embedding agent, represented by the following Formula 1, and has a molecular weight distribution satisfying the following condition X.

Formula 1: $\text{A-[(Gly-X-Y)}_n]_m\text{-B}$

In the formula, n X's each independently represent any amino acid, n Y's each independently represent any amino acid, m is an integer of 2 to 10, n is an integer of 3 to 100, A represents any amino acid or any amino acid sequence, and B represents any amino acid or any amino acid sequence.

Condition X: in a case where the molecular weight distribution is measured by gel permeation chromatography, a peak area of a maximum molecular weight is equal to or greater than 80% of the total peak area of all molecular weights.

[10] Method for embedding a cell sheet, comprising embedding a cell sheet in a polypeptide which is represented by the following Formula 1 and has a molecular weight distribution satisfying the following condition X.

Formula 1: $\text{A-[(Gly-X-Y)}_n]_m\text{-B}$

In the formula, n X's each independently represent any amino acid, n Y's each independently represent any amino acid, m is an integer of 2 to 10, n is an integer of 3 to 100, A represents any amino acid or any amino acid sequence, and B represents any amino acid or any amino acid sequence.

Condition X: in a case where the molecular weight distribution is measured by gel permeation chromatography, a peak area of a maximum molecular weight is equal to or greater than 80% of the total peak area of all molecular weights.

According to the cell sheet embedding agent, the cell sheet-containing composition, and the kit of the present invention, it is possible to stably transport a cell sheet at the time of transport at a low temperature, and the cell sheet can be collected from a cell sheet embedding agent in a simple manner after being transported.

**BRIEF DESCRIPTION OF THE DRAWINGS**

Fig. 1 shows a molecular weight distribution of recombinant gelatin.
Fig. 2 shows a molecular weight distribution of animal-derived natural gelatin.
Fig. 3 shows the shapes of a cell sheet embedded in CBE3 and a cell sheet containing no CBE3 that are obtained before and after shaking.
Fig. 4 shows the results of staining of a cell sheet embedded in CBE3 that are obtained before and after shaking.

**DESCRIPTION OF THE PREFERRED EMBODIMENTS**

Hereinafter, embodiments of the present invention will be specifically described.

<Cell sheet embedding agent>

The cell sheet embedding agent of the present invention is a cell sheet embedding agent containing a polypeptide which is represented by the following Formula 1 and has a molecular weight distribution satisfying the following condition X.

Formula 1: $A\text{-}[(Gly\text{-}X\text{-}Y)_n]_m\text{-}B$

In the formula, n X's each independently represent any amino acid, n Y's each independently represent any amino acid, m is an integer of 2 to 10, n is an integer of 3 to 100, A represents any amino acid or any amino acid sequence, and B represents any amino acid or any amino acid sequence.

Condition X: in a case where the molecular weight distribution is measured by gel permeation chromatography, a peak area of a maximum molecular weight is equal to or greater than 80% of the total peak area of all molecular weights.

The cell sheet embedding agent mentioned in the present specification is a substance which can perform an embedding treatment on a cell sheet. The embedding treatment is a treatment of bringing a cell sheet embedding agent into contact with a portion or the entirety of a surface of a cell sheet through immersion or the like and then coating a portion or the entirety of the surface of the cell sheet with the cell sheet embedding agent.

[Polypeptide]

By embedding a cell sheet in the polypeptide represented by Formula 1 in a solution state (under a condition that is not at a low temperature) and then reducing the temperature, the polypeptide is gelled and becomes in a state suitable for transport. From the viewpoint of preserving cells, the cells need to be transported at a low temperature. Furthermore, in the present invention, in a case where the polypeptide satisfies the condition X (in a case where the molecular weight distribution is measured by gel permeation chromatography, a peak area of a maximum molecular weight is equal to or greater than 80% of the total peak area of all molecular weights), gelation at a low temperature (4°C) and dissolution at room temperature (25°C) distinctly occur. As a result, at the time of transplanting the cell sheet, it is possible to collect the cell sheet from the cell sheet embedding agent in a simple manner at room temperature.

As described above, in the present invention, in a case where the molecular weight distribution is measured by gel permeation chromatography, a peak area of a maximum molecular weight is equal to or greater than 80% of the total peak area of all molecular weights. The peak area of the maximum molecular weight is more preferably equal to or higher than 85% and particularly preferably equal to or higher than 90% of the total peak area.

The molecular weight distribution can be measured by gel permeation chromatography by using high-performance liquid chromatography (HPLC) (AQUITY UPLC system Empower 2 manufactured by Waters Corporation) and a 100 mmol/L phosphate buffer (pH 6.8) as a buffer solution.

In Formula 1, m is an integer of 2 to 10. m preferably represents an integer of 3 to 5.

In Formula 1, n is an integer of 3 to 100. n is preferably an integer of 15 to 70, and more preferably an integer of 50 to 65.

The polypeptide represented by Formula 1 used in the present invention may be any of a recombinant polypeptide, a chemically synthesized polypeptide, and a natural polypeptide, as long as the molecular weight distribution thereof satisfies the condition X.

The chemically synthesized polypeptide means an artificially synthesized polypeptide. The synthesis of the polypeptide may be solid-phase synthesis or liquid-phase synthesis, and is preferably solid-phase synthesis. The solid-phase synthesis of a polypeptide is known to those skilled in the related art, and examples thereof include an Fmoc group synthesis method in which a Fluorenyl-Methoxy-Carbonyl group (Fmoc group) is used for protecting an amino group, a Boc group synthesis method in which a tert-Butyl Oxy Carbonyl group (Boc group) is used for protecting an amino group, and the like.

The polypeptide is preferably a recombinant polypeptide. In the present specification, the recombinant polypeptide represented by Formula 1 is referred to as recombinant gelatin. The recombinant gelatin will be described later in the present specification.

"1/IOB" value which is a hydrophilicity value of the polypeptide used in the present invention is preferably 0 to 1.0, more preferably 0 to 0.6, and even more preferably 0 to 0.4. IOB is an index of hydropathicity based on the organic conception diagram showing polarity/non-polarity of organic compounds that was suggested by Atsushi Fujita. Details of IOB are described, for example, in "Pharmaceutical Bulletin", vol. 2, 2, pp. 163-173 (1954), "Scope of Chemistry", vol. 11, 10, pp.719-725 (1957), "Fragrance Journal", vol. 50, pp. 79-82 (1981), and the like. In brief, methane ($CH_4$) is regarded as the origin of all organic compounds, all other compounds are regarded as derivatives of methane, and a certain numerical value is assigned to the number of carbon atoms, the substituent, the transformative portion, the ring, and the like of the compounds. The scores are added up so as to determine an organic value (OV) and an inorganic value (IV), and a graph is created by plotting the organic value on the X-axis and the inorganic value on the Y-axis. IOB in the organic conception diagram refers to a ratio of the inorganic value (IV) to the organic value (OV) in the organic conception diagram, that is, "inorganic value (IV)/organic value (OV)". For details of the organic conception diagram, see "New Edition of Organic Conception Diagram-Fundamentals and Applications-" (Yoshiki Koda et al., SANKYO SHUPPAN Co., Ltd., 2008). In the present specification, hydropathicity is represented by "1/IOB" value which is the reciprocal of IOB showing that the smaller the "1/IOB" value (the closer the "1/IOB" value to 0), the higher the hydrophilicity.

In a case where the "1/IOB" value of the polypeptide used in the present invention is within the above range, the hydrophilicity is high, and the water absorbing properties are improved.

A hydropathicity index of the polypeptide used in the present invention that is represented by a Grand average of hydropathicity (GRAVY) value is preferably equal to or lower than 0.3 and equal to or higher than -9.0, and more preferably equal to or lower than 0.0 and equal to or higher than -7.0. The Grand average of hydropathicity (GRAVY) value can be obtained by the methods in "Gasteiger E., Hoogland C., Gattiker A., Duvaud S., Wilkins M.R., Appel R.D., Bairoch A.; Protein Identification and Analysis Tools on the ExPASy Server;(In) John M. Walker (ed): The Proteomics Protocols Handbook, Humana Press (2005). pp. 571-607" and "Gasteiger E., Gattiker A., Hoogland C., Ivanyi I., Appel R.D., Bairoch A.; ExPASy: the proteomics server for in-depth protein knowledge and analysis.; Nucleic Acids Res. 31:3784-3788(2003)".

In a case where the GRAVY value of the polypeptide used in the present invention is within the above range, the hydrophilicity is high, and the water absorbing properties are improved.

[Recombinant gelatin]

The polypeptide used in the present invention is preferably recombinant gelatin.

As the recombinant gelatin, for example, it is possible to use those described in EP1014176, US6992172B, WO2004/85473A, WO2008/103041A, and the like. However, the recombinant gelatin is not limited thereto. As the recombinant gelatin used in the present invention, recombinant gelatin of the following aspect is preferable.

The recombinant gelatin exhibits excellent biocompatibility due to the performance inherent to the natural gelatin, does not carry a risk of causing bovine spongiform encephalopathy (BSE) because the recombinant gelatin is not derived from nature, and is excellently noninfectious.

The molecular weight of the recombinant gelatin is not particularly limited, but is preferably equal to or greater than 2,000 and equal to or smaller than 100,000 (equal to or greater than 2 kDa (kilodaltons) and equal to or smaller than 100 kDa), more preferably equal to or greater than 2,500 and equal to or smaller than 95,000 (equal to or greater than 2.5 kDa and equal to or smaller than 95 kDa), even more preferably equal to or greater than 5,000 and equal to or smaller than 90,000 (equal to or greater than 5 kDa and equal to or smaller than 90 kDa), and most preferably equal to or greater than 10,000 and equal to or smaller than 90,000 (equal to or greater than 10 kDa and equal to or smaller than 90 kDa).

It is preferable that the recombinant gelatin has a repeating sequence represented by Gly-X-Y characteristic of collagen. A plurality of sequences represented by Gly-X-Y may be the same as or different from each other. In Gly-X-Y, Gly represents glycine, and each of X and Y represents any amino acid (preferably any amino acid other than glycine). The sequence represented by Gly-X-Y characteristic of collagen is a partial structure extremely unique in the composition and sequence of the amino acid of gelatin·collagen compared to other proteins. Glycine takes up about 1/3 of this portion and is one of the three repeating amino acids in the amino acid sequence. Glycine is the simplest amino acid, and the disposition of the molecular chain thereof is restricted less. Furthermore, glycine makes a big contribution to the regeneration of a helix structure at the time of gelation. The amino acids represented by X and Y contain a large amount of imino acids (proline and oxyproline), and the content of the imino acids is preferably 10% to 45% of the total content of the amino acids. In the sequence of the recombinant gelatin, the proportion of amino acids constituted with the repeating structure of Gly-X-Y is preferably equal to or higher than 80%, more preferably equal to or higher than 95%, and most preferably equal to or higher than 99%.

Generally, gelatin contains charged polar amino acids and uncharged polar amino acids at 1:1. The polar amino acids specifically refer to cysteine, aspartic acid, glutamic acid, histidine, lysine, asparagine, glutamine, serine, threonine, tyrosine, and arginine. Among these, uncharged polar amino acids refer to cysteine, asparagine, glutamine, serine, threonine, and tyrosine. In the recombinant gelatin used in the present invention, the proportion of the polar amino acids in all the constituent amino acids is 10% to 40% and preferably 20% to 30%. The proportion of the uncharged amino acids in the polar amino acids is preferably equal to or higher than 5% and less than 20%, and more preferably equal to or higher than 5% and less than 10%. Furthermore, it is preferable that gelatin does not contain one amino acid and preferably does not contain two or more amino acids among serine, threonine, asparagine, tyrosine, and cysteine in the sequence thereof.

Generally, regarding polypeptides, minimal amino acid sequence functioning as cell adhesion signals are known (for example, "Pathophysiology" published from Nagai Publishing Co., Ltd., Vol. 9, No. 7 (1990), p. 527). The recombinant gelatin used in the present invention may have two or more cell adhesion signals in one molecule. Specifically, as such sequences, an RGD sequence, an LDV sequence, an REDV sequence, a YIGSR sequence, a PDSGR sequence, an RYVVLPR sequence, an LGTIPG sequence, an RNIAEIIKDI sequence, an IKVAV sequence, an LRE sequence, a DGEA sequence, and an HAV sequence represented by one-letter notation for amino acids are preferable, because these sequences enable the adhesion of many kinds of cells. Among these, an RGD sequence, a YIGSR sequence, a PDSGR sequence, an LGTIPG sequence, an IKVAV sequence, and an HAV sequence are more preferable, and an RGD sequence is particularly preferable. Among RGD sequences, an ERGD sequence is preferable.

Regarding the disposition of the RGD sequence in the recombinant gelatin used in the present invention, it is preferable that the number of amino acids between RGD sequences varies in a range of 0 to 100 and preferably varies in a range of 25 to 60.

The number of minimal amino acid sequences described above contained in one molecule of the protein is preferably 3 to 50, more preferably 4 to 30, particularly preferably 5 to 20, and most preferably 12.

In the recombinant gelatin used in the present invention, the ratio of the RGD motif to the total number of amino acids is preferably at least 0.4%. In a case where the recombinant gelatin contains 350 or more amino acids, it is preferable that each stretch of the 350 amino acids contains at least one RGD motif. The ratio of the RGD motif to the total number of amino acids is more preferably at least 0.6%, even more preferably at least 0.8%, still more preferably at least 1.0%, particularly preferably at least 1.2%, and most preferably at least 1.5%. The number of RGD motifs in the recombinant peptide is preferably at least 4, more preferably 6, even more preferably 8, and particularly preferably equal to or greater than 12 and equal to or smaller than 16 per 250 amino acids. The ratio of 0.4% of the RGD motif means that the recombinant gelatin contains at least one RGD sequence per 250 amino acids. The number of RGD motifs is an integer. Therefore, in order to satisfy the characteristic of 0.4%, gelatin constituted with 251 amino acids has to contain at least two RGD sequences. The recombinant gelatin of the present invention preferably contains at least two RGD sequences per 250 amino acids, more preferably contains at least three RGD sequences per 250 amino acids, and even more preferably contains at least four RGD sequences per 250 amino acids. In another aspect, the recombinant gelatin of the present invention contains at least four RGD motifs, preferably contains six RGD motifs, more preferably contains eight RGD motifs, and even more preferably contains twelve to sixteen RGD motifs.

The recombinant gelatin may be partially hydrolyzed.

It is more preferable that the polypeptide used in the present invention is represented by the following Formula 2.

Formula 2: Gly-Ala-Pro-[(Gly-X-Y)$_{63}$]$_3$-Gly

In the formula, 63 X's each independently represent any amino acid, 63 Y's each independently represent any amino acid, and 63 sequences represented by Gly-X-Y may be the same as or different from each other.

It is preferable that a plurality of sequence units of naturally occurring collagen are bonded to the repeating unit. The naturally occurring collagen is not limited as long as it exists in the nature. As the naturally occurring collagen, type I, type II, type III, type IV, or type V collagen is preferable, and type I, type II, or type III collagen is more preferable. According to another aspect, the collagen is preferably derived from human beings, cows, pigs, mice, or rats, and more preferably derived from human beings.

The isoelectric point of the recombinant gelatin used in the present invention is preferably 5 to 10, more preferably 6 to 10, and even more preferably 7 to 9.5. The isoelectric point of the recombinant gelatin can be determined by measuring pH after allowing a 1% by mass gelatin solution and cation and anion exchange resins to pass through a mixed crystal column as described in isoelectric focusing electrophoresis (see Maxey, C. R. (1976); Phitogr. Gelatin 2, Editor Cox, P. J. Academic, London, Engl.).

It is preferable that the recombinant gelatin is deaminated.

It is preferable that the recombinant gelatin does not have a telopeptide.

It is preferable that the recombinant gelatin is substantially a pure polypeptide prepared by nucleic acids encoding amino acid sequences.

It is particularly preferable that the recombinant gelatin has (1) amino acid sequence described in SEQ ID NO: 1 or (2) amino acid sequence which shares a sequence identity equal to or higher than 80% (preferably equal to or higher than 90%, more preferably equal to or higher than 95%, and particularly preferably equal to or higher than 98%) with the amino acid sequence described in SEQ ID NO: 1 and has biocompatibility.

The biocompatibility means a property of not inducing a markedly harmful response such as a prolonged and chronic inflammatory response by contact with a biological body.

It is most preferable that the recombinant gelatin has the amino acid sequence described in SEQ ID NO: 1.

In the present invention, the sequence identity refers to a value calculated by the following formula.

$$\% \text{ Sequence identity} = [(\text{number of same residues})/(\text{alignment length})] \times 100$$

The sequence identity shared between two amino acid sequences can be determined by any method known to those skilled in the related art by using a Basic Local Alignment Search Tool (BLAST) program (J. Mol. Biol. 215:403-410, 1990) and the like.

The recombinant gelatin may have an amino acid sequence which is obtained by the deletion, substitution, or addition of one or several amino acids in the amino acid sequence described in SEQ ID NO: 1 and has biocompatibility.

"One or several amino acids" in "amino acid sequence which is obtained by the deletion, substitution, or addition of one or several amino acids" preferably means 1 to 20 amino acids, more preferably means 1 to 10 amino acids, even more preferably means 1 to 5 amino acids, and particularly preferably means 1 to 3 amino acids.

The recombinant gelatin can be manufactured by gene recombination technologies known to those skilled in the related art. For example, the recombinant gelatin can be manufactured based on the methods described in EP1014176A2, US6992172B, WO2004/85473A, WO2008/103041A, and the like. Specifically, genes encoding an amino acid sequence of a predetermined recombinant gelatin are obtained and incorporated into an expression vector, thereby preparing a recombinant expression vector. The recombinant expression vector is introduced into an appropriate host, thereby preparing a transformant. By culturing the obtained transformant in an appropriate medium, recombinant gelatin is produced. Therefore, by collecting the recombinant gelatin produced from the culture, the recombinant gelatin used in the present invention can be prepared.

[Form of cell sheet embedding agent]

The form of the cell sheet embedding agent of the present invention is not particularly limited. The cell sheet embedding agent can take any form as long as it contains a polypeptide which is represented by Formula 1 and has a molecular weight distribution satisfying the condition X. For example, the cell sheet embedding agent may be in any of the forms of a solution (aqueous solution or the like), a suspension, powder, and gel. In a case where the cell sheet embedding agent is in the form of powder, gel, or the like, the agent can be used by being dissolved in a solvent such as water at the time of use.

The content of the polypeptide in the cell sheet embedding agent of the present invention is not particularly limited, but is generally 0.1% by mass to 100% by mass, and preferably 0.5% by mass to 100% by mass.

The cell sheet to which the cell sheet embedding agent of the present invention is applied will be described later in the present specification.

<Cell sheet-containing composition>

The present invention also relates to a cell sheet-containing composition containing a cell sheet and the cell sheet embedding agent of the present invention, in which the cell sheet is embedded in the cell sheet embedding agent.

[Cell sheet]

In the present invention, the cell sheet means a sheet containing cells as a main component. The cell sheet is a substance in which cells form a sheet by being linked to each other. The constitution of the cell sheet is not particularly limited as long as the cell sheet has a sheet shape. The cell sheet may be any of a single-layer cell sheet, a sheet constituted with two or more layers formed of cells, and a sheet formed of three-dimensionally cultured cells.

The cells may be linked to each other directly and/or through an interpositional substance. The interpositional substance is not particularly limited as long as it is a substance which can link the cells to each other in at least a mechanical way, and examples thereof include the extracellular matrix and the like. The interpositional substance is preferably derived from cells and particularly derived from the cells constituting the cell sheet. The cells are linked to each other in at least a mechanical way, but the cells may also be linked to each other functionally, for example, chemically or electrically.

The cell sheet in the present invention contains any cells capable of forming a cell sheet. Examples of the cells are not particularly limited and include myocardial cells, myoblasts (for example, skeletal myoblasts), fibroblasts, synovial cells, epithelial cells, endothelial cells, and the like. Among these, myocardial cells and skeletal myoblasts are preferable. As the cells, it is possible to use cells derived from any organism which can be treated using the cell sheet. The organism is not particularly limited, and examples thereof include human beings, non-human primates (monkey and the like), dogs, cats, pigs, horses, goats, lambs, rats, mice, hamsters, and the like. One kind of cells may be used singly, or two or more kinds of cells may be used.

The cell sheet can be manufactured by known cell sheet manufacturing methods or methods equivalent thereto. For example, the cell sheet may be manufactured by culturing cells in a culture plate, allowing the cells to form a sheet, and then collecting the sheet from the culture plate. As described above, the method for manufacturing the cell sheet is not particularly limited, but for example, the cell sheet can be manufactured by the method described in JP2010-81829A.

Typically, the cell sheet is manufactured by a step of seeding cells in a culture solution and a step of forming a cell sheet by culturing the cells.

The cell culture can be performed under the conditions that are generally adopted in the technical field of the present invention. For example, typically, the cells are cultured under the conditions of 37°C and 5% $CO_2$.

[Manufacturing of cell sheet-containing composition]

The cell sheet-containing composition can be manufactured by embedding the cell sheet in the cell sheet embedding agent of the present invention. The embedding method is not particularly limited. By adding the embedding agent (preferably a solution) of the present invention to a container containing the cell sheet and cooling the container at a low temperature (for example, 2°C to 12°C), the solution can be gelled. In this way, the cell sheet-containing composition in which the cell sheet is embedded in the cell sheet embedding agent can be manufactured.

<Kit>

According to the present invention, there is also provided a kit including the cell sheet and the cell sheet embedding agent of the present invention. Details and preferred aspects of the cell sheet and the cell sheet embedding agent are as described above in the present specification. The kit may further include a container for performing an embedding treatment, an instruction manual, and the like.

The present invention will be more specifically described based on the following examples, but the present invention is not limited to the examples.

Examples

(1) Recombinant gelatin

As recombinant gelatin, the following CBE3 (described in WO2008/103041A) was prepared.

CBE3:

Molecular weight: 51.6 kD

Structure: $GAP[(GXY)_{63}]_3G$

Number of amino acids: 571

Number of RGD sequences: 12

Content of imino acid: 33%

Approximately 100% of the amino acids are repeating GXY structures. The amino acid sequence of CBE3 does not contain serine, threonine, asparagine, tyrosine, and cysteine. CBE3 has an ERGD sequence.

Isoelectric point: 9.34

GRAVY value: -0.682

1/IOB value: 0.323

Amino acid sequence (SEQ ID NO: 1 in sequence listing) (same as SEQ ID NO: 3 in WO2008/103041A except that the terminal X is revised to "P")

GAP(GAPGLQGAPGLQGMPGERGAAGLPGPKGERGDAGPKGADGAPGAPGLQGMPG
ERGAAGLPGPKGERGDAGPKGADGAPGKDGVRGLAGPIGPPGERGAAGLPGPKGER
GDAGPKGADGAPGKDGVRGLAGPIGPPGPAGAPGAPGLQGMPGERGAAGLPGPKGE
RGDAGPKGADGAPGKDGVRGLAGPP)3G

(2) Measurement of molecular weight distribution of recombinant gelatin

The molecular weight distribution of the recombinant gelatin CBE3 (Example 1) explained in (1) described above was measured by gel permeation chromatography (GPC). The molecular weight distribution was measured using HPLC (AQUITY UPLC system Empower 2 manufactured by Waters Corporation) and a 100 mmol/L phosphate buffer (pH 6.8) as a buffer solution. By using these, the relativity of the molecular weight distribution of the recombinant gelatin was measured.

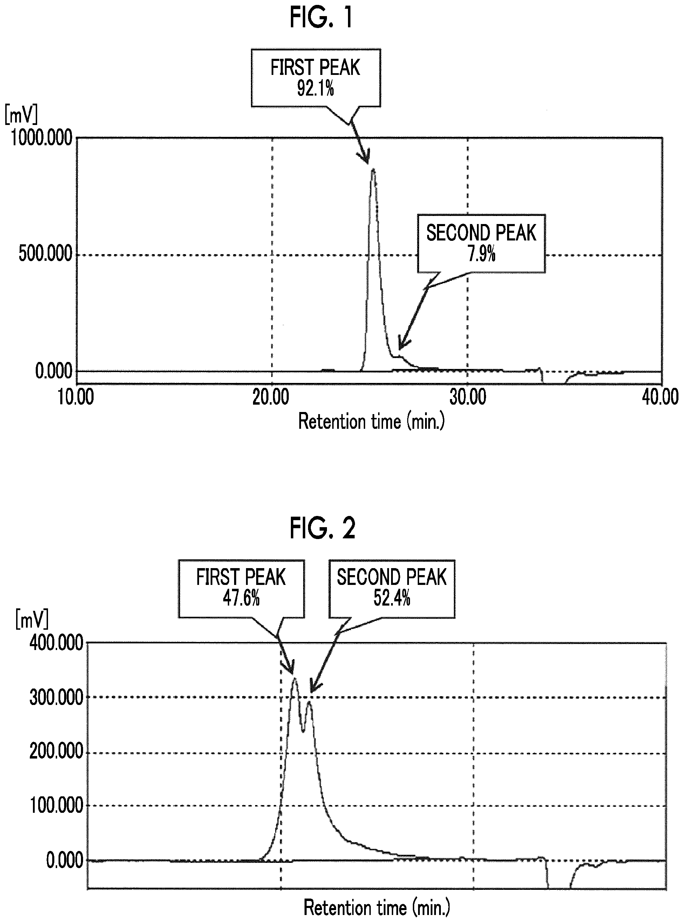

The results are shown in Fig. 1. CBE3 had two molecular weight peaks. From the area ratios of the two peaks, the occupancy ratio thereof in all peaks was calculated. As a result, the occupancy ratio of the first peak and the second peak was 92.1% and 7.9% respectively. From this result, it was understood that CBE3 has a molecular weight peak taking up 92% of all peaks as a peak having a high occupancy ratio.

(3) Measurement of molecular weight distribution of animal-derived natural gelatin.

For comparison, the molecular weight distribution of the animal-derived natural gelatin (Comparative Example 1) was measured by GPC in the same manner as in (2) described above. The results are shown in Fig. 2. The animal-derived natural gelatin had two molecular weight peaks. From the area ratios of the two peaks, the occupancy ratio thereof in all peaks was calculated. As a result, the occupancy ratio of the first peak and the second peak was 47.6% and 52.4% respectively. From this result, it was understood that the animal-derived natural gelatin has a molecular weight peak taking up 52% of all peaks as a peak having a high occupancy ratio.

(4) Performance test for embedding agent (gelling rate)

In order for an embedding agent to be used for transporting a cell sheet, it is important for the embedding agent to demonstrate performance of exhibiting a high gelling rate at a low temperature yet performing, as a reversible reaction, swift returning to a liquid form at room temperature. Therefore, the gelling rate at a low temperature and how fast the embedding agent returns to a liquid form at room temperature were tested at a concentration of 1% by mass.

As a result, for CBE3 in Example 1, a phenomenon was observed in which the sample is rapidly gelled and the fluidity of the liquid is rapidly reduced in a case where the sample is moved to an environment with a temperature of 4°C, and the completely gelled sample swiftly returns to a liquid form in a case where the sample is moved to an environment with room temperature of 25°C.

Specifically, by being moved to an environment with a temperature of 4°C, CBE3 started to be gelled after 40 minutes and then turned into substantially stable gel after 50 minutes. The gel had hardness and was not crushed even being pressed by a hand. In a case where the gel was returned to room temperature of 25°C, the gel completely returned to the liquid form within 40 minutes.

In contrast, although the animal-derived natural gelatin of Comparative Example 1 started to be gelled by being moved to an environment with a temperature of 4°C, the gelling rate was slow and lower than that of CBE3. Furthermore, as a result of moving the completely gelled sample to an environment with room temperature of 25°C so as to test how fast the sample returns to the liquid form, the animal-derived natural gelatin of Comparative Example 1 slowly returned to the liquid form compared to CBE3 in Example 1.

Specifically, although the animal-derived natural gelatin started to be gelled after 60 minutes by being moved to an environment with a temperature of 4°C, the gelatin remains as soft gel even after 90 minutes. Even after then, the gel did not turn into gel having hardness and remained as soft gel simply crushed by being pressed by a hand. In a case where the gel-like sample was returned to room temperature of 25°C, it took 70 minutes for the animal-derived natural gelatin to completely return to the liquid form.

(5) Preparation of cell sheet embedded in CBE3

Human skeletal myoblasts at $6.0 \times 10^7$ cells were suspended in 10 mL of a DMEM/F12 medium containing 20% (v/v) human serum, and the cells were seeded in a 10 cm dish temperature-responsive culture plate (UpCell (registered trademark): manufactured by CellSeed Inc.). The cells were cultured overnight under the conditions of 37°C and 5% $CO_2$ so as to make a cell sheet, and the cell sheet was peeled at room temperature. At room temperature, 10 mL of HBSS+ (manufactured by Thermo Fisher Scientific Inc.) containing 1% by mass CBE3 was added to a temperature-responsive culture plate and cooled for 1 hour at 2°C to 8°C so as to make a gel, thereby preparing a cell sheet embedded in CBE3. HBSS is the abbreviation of Hanks' Balanced Salt solution.

(6) Shaking evaluation

(6-1)

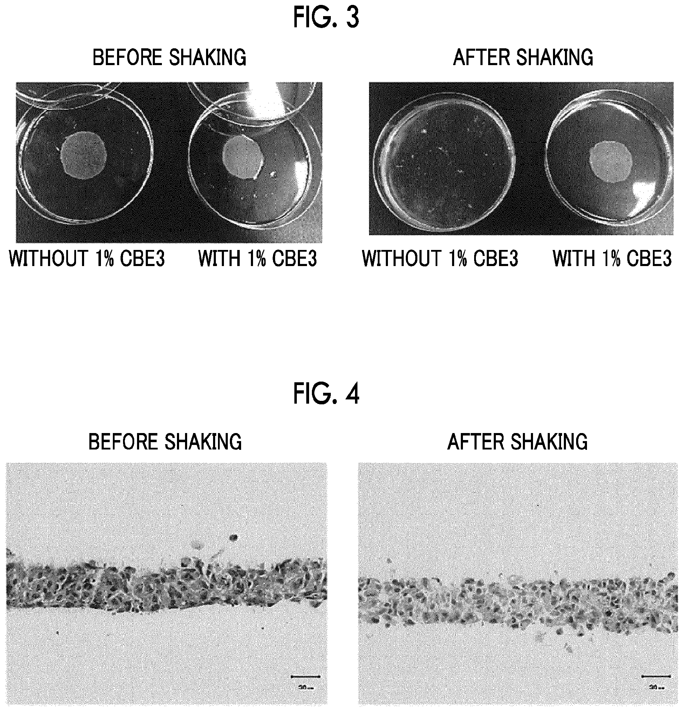

The cell sheet embedded in CBE3 prepared in (5) described above was placed on a desktop shaker (NR-3, manufactured by TAITEC CORPORATION) and shaken for 2 days at 2°C to 8°C by setting the rotation speed to be 100 rpm (rotation per minute). As a control, HBSS+ containing no CBE3 was added to the cell sheet prepared using the 10 cm dish temperature-responsive culture plate, and shaken in the same manner. After shaking, the cell sheet embedded in CBE3 was allowed to stand at room temperature such that the gel was melted, and the cell sheet was collected. Fig. 3 shows the shapes obtained before and after shaking.
In a case where HBSS+ containing no CBE3 was added, the cell sheet was broken after shaking. In contrast, in a case where the cell sheet was embedded in HBSS+ containing 1% by mass CBE3, the cell sheet was not broken even after shaking.

(6-2)

HBSS+ containing 1% by mass CBE3 was added to a just prepared cell sheet and shaken, and then the cell sheet was collected. The collected cell sheet was fixed overnight in a 10% by mass neutral buffered formalin, and the cell sheet was embedded in paraffin. The embedded sample was sliced, deparaffinized, and then stained with Carrazzi's hematoxylin and eosin. The results are shown in Fig. 4.
In a case where the cell sheet was embedded in HBSS+ containing 1% by mass CBE3 added thereto, even after shaking, the cells in the cell sheet survived just like the cells at the time of preparation, and it was confirmed that the cell sheet can sufficiently demonstrate its performance.

Sequence listing

International application 16F02134 accepted based on Patent Cooperation Treaty Cell sheet embedding agent, Cell sheet-containing JP17012284 20170327---- 00040120451700658041Normal20170327115243201703081113320050 P1AP1 01 16 O.app

**Claims**

1. A cell sheet embedding agent comprising:

   a polypeptide which is represented by the following Formula 1 and has a molecular weight distribution satisfying the following condition X,

   Formula 1:  $\text{A-[(Gly-X-Y)}_n]_m\text{-B}$

   in the formula, n X's each independently represent any amino acid, n Y's each independently represent any amino acid, m is an integer of 2 to 10, n is an integer of 3 to 100, A represents any amino acid or any amino acid sequence, and B represents any amino acid or any amino acid sequence,
   Condition X: in a case where the molecular weight distribution is measured by gel permeation chromatography, a peak area of a maximum molecular weight is equal to or greater than 80% of the total peak area of all molecular weights.

2. The cell sheet embedding agent according to claim 1,
   wherein the polypeptide is recombinant gelatin.

**3.** The cell sheet embedding agent according to claim 1 or 2,
wherein the polypeptide is a polypeptide represented by the following Formula 2,

Formula 2: $\text{Gly-Ala-Pro-[(Gly-X-Y)}_{63}]_3\text{-Gly}$

in the formula, 63 X's each independently represent any amino acid, 63 Y's each independently represent any amino acid, and 63 sequences represented by Gly-X-Y may be the same as or different from each other.

**4.** The cell sheet embedding agent according to any one of claims 1 to 3,
wherein the polypeptide has (1) amino acid sequence described in SEQ ID NO: 1 or (2) amino acid sequence which shares a sequence identity equal to or higher than 80% with the amino acid sequence described in SEQ ID NO: 1 and has biocompatibility.

**5.** The cell sheet embedding agent according to any one of claims 1 to 4,
wherein the polypeptide has the amino acid sequence described in SEQ ID NO: 1.

**6.** A cell sheet-containing composition comprising:

a cell sheet; and
the cell sheet embedding agent according to any one of claims 1 to 5,
wherein the cell sheet is embedded in the cell sheet embedding agent.

**7.** A kit comprising:

a cell sheet; and
the cell sheet embedding agent according to any one of claims 1 to 5.

## FIG. 1

FIRST PEAK
92.1%

SECOND PEAK
7.9%

Retention time (min.)

## FIG. 2

FIRST PEAK
47.6%

SECOND PEAK
52.4%

Retention time (min.)

# FIG. 3

BEFORE SHAKING

AFTER SHAKING

WITHOUT 1% CBE3    WITH 1% CBE3        WITHOUT 1% CBE3    WITH 1% CBE3

# FIG. 4

BEFORE SHAKING

AFTER SHAKING

**INTERNATIONAL SEARCH REPORT**

| International application No. |
| --- |
| PCT/JP2017/012284 |

A. CLASSIFICATION OF SUBJECT MATTER
*C07K14/78*(2006.01)i, *A61L27/00*(2006.01)i, *C12M1/00*(2006.01)i, *C12N5/07*
(2010.01)i, *C12N15/09*(2006.01)i

According to International Patent Classification (IPC) or to both national classification and IPC

B. FIELDS SEARCHED

Minimum documentation searched (classification system followed by classification symbols)
C07K14/78, A61L27/00, C12M1/00, C12N5/07, C12N15/09

Documentation searched other than minimum documentation to the extent that such documents are included in the fields searched
Jitsuyo Shinan Koho          1922–1996   Jitsuyo Shinan Toroku Koho    1996–2017
Kokai Jitsuyo Shinan Koho    1971–2017   Toroku Jitsuyo Shinan Koho    1994–2017

Electronic data base consulted during the international search (name of data base and, where practicable, search terms used)
JSTPlus/JMEDPlus/JST7580(JDreamIII), CAplus/MEDLINE/EMBASE/BIOSIS(STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y | JP 2011-172925 A  (Terumo Corp.),<br>08 September 2011 (08.09.2011),<br>claims; example 1<br>(Family: none) | 1,6-7<br>1-7 |
| Y | JP 8-23968 A  (Gunze Ltd.),<br>30 January 1996 (30.01.1996),<br>claims; paragraph [0007]; examples<br>(Family: none) | 1-7 |
| Y | Yoshimi OYABU et al., "Gel-ka Ondo ga Kojo<br>shita Gelatin ni yoru Saibo Seat no Hogo",<br>Regenerative Medicine, 01 February 2016 (01.02.<br>2016), vol.15, Suppl 2016, page 307, P-01-078,<br>entire text | 1-7 |

| ☒  Further documents are listed in the continuation of Box C. | ☐  See patent family annex. |
| --- | --- |

| * Special categories of cited documents: | "T" later document published after the international filing date or priority |
| --- | --- |

* Special categories of cited documents:
"A" document defining the general state of the art which is not considered    to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed

"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search<br>25 April 2017 (25.04.17) | Date of mailing of the international search report<br>09 May 2017 (09.05.17) |
| --- | --- |
| Name and mailing address of the ISA/<br>Japan Patent Office<br>3-4-3,Kasumigaseki,Chiyoda-ku,<br>Tokyo 100-8915,Japan | Authorized officer<br><br>Telephone No. |

Form PCT/ISA/210 (second sheet) (January 2015)

**INTERNATIONAL SEARCH REPORT**

International application No.

PCT/JP2017/012284

C (Continuation).    DOCUMENTS CONSIDERED TO BE RELEVANT

| Category* | Citation of document, with indication, where appropriate, of the relevant passages | Relevant to claim No. |
|---|---|---|
| Y | JP 2010-519251 A (Fujifilm Manufacturing Europe B.V.), 03 June 2010 (03.06.2010), claims; paragraphs [0008], [0012], [0029], [0030]; examples & WO 2008/103041 A1 claims; page 2, lines 5 to 21; page 3, lines 1 to 3; page 7, line 29 to page 8, line 15; examples & US 2010/0119574 A1     & EP 1961411 A1 | 1-7 |

Form PCT/ISA/210 (continuation of second sheet) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- JP 2010081829 A
- WO 2008103041 A
- EP 1014176 A
- US 6992172 B
- WO 200485473 A
- EP 1014176 A2
- JP 17012284 A

### Non-patent literature cited in the description

- *Pharmaceutical Bulletin,* 1954, vol. 2 (2), 163-173
- *Scope of Chemistry,* 1957, vol. 11 (10), 719-725
- *Fragrance Journal,* 1981, vol. 50, 79-82
- **YOSHIKI KODA et al.** New Edition of Organic Conception Diagram-Fundamentals and Applications. SANKYO SHUPPAN Co., Ltd, 2008
- Protein Identification and Analysis Tools on the ExPASy Server. **GASTEIGER E. ; HOOGLAND C. ; GATTIKER A. ; DUVAUD S. ; WILKINS M.R. ; APPEL R.D. ; BAIROCH A.** The Proteomics Protocols Handbook. Humana Press, 2005, 571-607
- **GASTEIGER E. ; GATTIKER A. ; HOOGLAND C. ; IVANYI I. ; APPEL R.D. ; BAIROCH A.** ExPASy: the proteomics server for in-depth protein knowledge and analysis. *Nucleic Acids Res.,* 2003, vol. 31, 3784-3788
- Pathophysiology. Nagai Publishing Co., Ltd, 1990, vol. 9, 527
- **MAXEY, C. R.** Phitogr. Gelatin 2. Academic, 1976
- *J. Mol. Biol.,* 1990, vol. 215, 403-410

SEQUENCE LISTING

<110>  FUJIFILM Corporation

<120>  A cell sheet-embedding agent, a cell sheet-containing composition and a kit

<130>\201@16F02134

<160>  10

<170>  PatentIn version 3.5

<210>  1
<211>  571
<212>  PRT
<213>  Recombinant

<400>  1

Gly Ala Pro Gly Ala Pro Gly Leu Gln Gly Ala Pro Gly Leu Gln Gly
1               5                   10                  15
Met Pro Gly Glu Arg Gly Ala Ala Gly Leu Pro Gly Pro Lys Gly Glu
                20                  25                  30
Arg Gly Asp Ala Gly Pro Lys Gly Ala Asp Gly Ala Pro Gly Ala Pro
            35                  40                  45
Gly Leu Gln Gly Met Pro Gly Glu Arg Gly Ala Ala Gly Leu Pro Gly
        50                  55                  60
Pro Lys Gly Glu Arg Gly Asp Ala Gly Pro Lys Gly Ala Asp Gly Ala
65                  70                  75                  80
Pro Gly Lys Asp Gly Val Arg Gly Leu Ala Gly Pro Ile Gly Pro Pro
                85                  90                  95
Gly Glu Arg Gly Ala Ala Gly Leu Pro Gly Pro Lys Gly Glu Arg Gly
                100                 105                 110
Asp Ala Gly Pro Lys Gly Ala Asp Gly Ala Pro Gly Lys Asp Gly Val
            115                 120                 125
Arg Gly Leu Ala Gly Pro Ile Gly Pro Pro Gly Pro Ala Gly Ala Pro
        130                 135                 140
Gly Ala Pro Gly Leu Gln Gly Met Pro Gly Glu Arg Gly Ala Ala Gly
145                 150                 155                 160
Leu Pro Gly Pro Lys Gly Glu Arg Gly Asp Ala Gly Pro Lys Gly Ala
                165                 170                 175
Asp Gly Ala Pro Gly Lys Asp Gly Val Arg Gly Leu Ala Gly Pro Pro
                180                 185                 190
Gly Ala Pro Gly Leu Gln Gly Ala Pro Gly Leu Gln Gly Met Pro Gly
            195                 200                 205
Glu Arg Gly Ala Ala Gly Leu Pro Gly Pro Lys Gly Glu Arg Gly Asp
        210                 215                 220
Ala Gly Pro Lys Gly Ala Asp Gly Ala Pro Gly Ala Pro Gly Leu Gln
225                 230                 235                 240
Gly Met Pro Gly Glu Arg Gly Ala Ala Gly Leu Pro Gly Pro Lys Gly
                245                 250                 255
Glu Arg Gly Asp Ala Gly Pro Lys Gly Ala Asp Gly Ala Pro Gly Lys
                260                 265                 270
Asp Gly Val Arg Gly Leu Ala Gly Pro Ile Gly Pro Pro Gly Glu Arg
            275                 280                 285
Gly Ala Ala Gly Leu Pro Gly Pro Lys Gly Glu Arg Gly Asp Ala Gly
        290                 295                 300
Pro Lys Gly Ala Asp Gly Ala Pro Gly Lys Asp Gly Val Arg Gly Leu
305                 310                 315                 320
Ala Gly Pro Ile Gly Pro Pro Gly Pro Ala Gly Ala Pro Gly Ala Pro
                325                 330                 335
Gly Leu Gln Gly Met Pro Gly Glu Arg Gly Ala Ala Gly Leu Pro Gly
                340                 345                 350
Pro Lys Gly Glu Arg Gly Asp Ala Gly Pro Lys Gly Ala Asp Gly Ala
            355                 360                 365
Pro Gly Lys Asp Gly Val Arg Gly Leu Ala Gly Pro Pro Gly Ala Pro
        370                 375                 380
Gly Leu Gln Gly Ala Pro Gly Leu Gln Gly Met Pro Gly Glu Arg Gly
385                 390                 395                 400
Ala Ala Gly Leu Pro Gly Pro Lys Gly Glu Arg Gly Asp Ala Gly Pro

```
                    405                      410                      415
       Lys Gly Ala Asp Gly Ala Pro Gly Ala Pro Gly Leu Gln Gly Met Pro
                        420                  425                  430
       Gly Glu Arg Gly Ala Ala Gly Leu Pro Gly Pro Lys Gly Glu Arg Gly
                    435                  440                  445
       Asp Ala Gly Pro Lys Gly Ala Asp Gly Ala Pro Gly Lys Asp Gly Val
            450                  455                  460
       Arg Gly Leu Ala Gly Pro Ile Gly Pro Pro Gly Glu Arg Gly Ala Ala
       465                  470                  475                  480
       Gly Leu Pro Gly Pro Lys Gly Glu Arg Gly Asp Ala Gly Pro Lys Gly
                        485                  490                  495
       Ala Asp Gly Ala Pro Gly Lys Asp Gly Val Arg Gly Leu Ala Gly Pro
                    500                  505                  510
       Ile Gly Pro Pro Gly Pro Ala Gly Ala Pro Gly Ala Pro Gly Leu Gln
                    515                  520                  525
       Gly Met Pro Gly Glu Arg Gly Ala Ala Gly Leu Pro Gly Pro Lys Gly
            530                  535                  540
       Glu Arg Gly Asp Ala Gly Pro Lys Gly Ala Asp Gly Ala Pro Gly Lys
       545                  550                  555                  560
       Asp Gly Val Arg Gly Leu Ala Gly Pro Pro Gly
                        565                  570
```

```
<210>  2
<211>  4
<212>  PRT
<213>  Artificial Sequence
<220>
<223>  Description of Artificial Sequence: adhesive sequence
<400>  2
Arg Glu Asp Val
1

<210>  3
<211>  5
<212>  PRT
<213>  Artificial Sequence
<220>
<223>  Description of Artificial Sequence: adhesive sequence
<400>  3
Tyr Ile Gly Ser Arg
1               5

<210>  4
<211>  5
<212>  PRT
<213>  Artificial Sequence
<220>
<223>  Description of Artificial Sequence: adhesive sequence
<400>  4
Pro Asp Ser Gly Arg
1               5

<210>  5
<211>  7
<212>  PRT
<213>  Artificial Sequence
<220>
<223>  Description of Artificial Sequence: adhesive sequence
<400>  5
Arg Tyr Val Val Leu Pro Arg
1               5

<210>  6
<211>  6
<212>  PRT
<213>  Artificial Sequence
<220>
<223>  Description of Artificial Sequence: adhesive sequence
```

```
<400>  6
Leu Gly Thr Ile Pro Gly
1               5
<210>  7
<211>  10
<212>  PRT
<213>  Artificial Sequence
<220>
<223>  Description of Artificial Sequence: adhesive sequence
<400>  7
Arg Asn Ile Ala Glu Ile Ile Lys Asp Ile
1               5                   10
<210>  8
<211>  5
<212>  PRT
<213>  Artificial Sequence
<220>
<223>  Description of Artificial Sequence: adhesive sequence
<400>  8
Ile Lys Val Ala Val
1               5
<210>  9
<211>  4
<212>  PRT
<213>  Artificial Sequence
<220>
<223>  Description of Artificial Sequence: adhesive sequence
<400>  9
Asp Gly Glu Ala
1
<210>  10
<211>  4
<212>  PRT
<213>  Artificial Sequence
<220>
<223>  Description of Artificial Sequence: adhesive sequence
<400>  10
Glu Arg Gly Asp
1
```